(19) Europäisches Patentamt
European Patent Office
Office européen des brevets

(11) EP 2 194 384 B1

(12) **EUROPÄISCHE PATENTSCHRIFT**

(45) Veröffentlichungstag und Bekanntmachung des Hinweises auf die Patenterteilung:
**01.11.2017 Patentblatt 2017/44**

(21) Anmeldenummer: **10003169.9**

(22) Anmeldetag: **13.12.2001**

(51) Int Cl.:
*A61M 1/16* (2006.01)     *A61M 1/14* (2006.01)
*G01N 33/84* (2006.01)     *A61M 1/34* (2006.01)
*A61M 1/36* (2006.01)

(54) **Verfahren zur Ermittlung der Ionenkonzentration des Blutes**

Method for determining the ion concentration of blood

Méthode pour la détermination de la concentration des ions dans le sang

(84) Benannte Vertragsstaaten:
**AT BE CH CY DE DK ES FI FR GB GR IE IT LI LU MC NL PT SE TR**

(30) Priorität: **22.12.2000 DE 10064179**
**23.03.2001 DE 10114283**

(43) Veröffentlichungstag der Anmeldung:
**09.06.2010 Patentblatt 2010/23**

(62) Dokumentnummer(n) der früheren Anmeldung(en) nach Art. 76 EPÜ:
**01129707.4 / 1 217 379**

(73) Patentinhaber: **Fresenius Medical Care Deutschland GmbH**
**61352 Bad Homburg (DE)**

(72) Erfinder:
• **Nier, Volker, Dr.**
**61203 Reichelsheim (DE)**
• **Krämer, Matthias, Dr.**
**61381 Friedrichsdorf (DE)**

(74) Vertreter: **Laufhütte, Dieter et al**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
EP-A- 0 330 892      EP-A- 0 898 976
EP-A- 0 952 453      WO-A-91/06326
US-A- 3 934 977      US-A- 4 618 587
US-A- 4 724 216      US-A- 5 714 060

**Beschreibung**

Die vorliegende Erfindung betrifft ein Verfahren zur Ermittlung der Konzentration eines in einem Komplex gebundenen Ions, Atoms oder Moleküls.

Zahlreiche Ionen, Atome und Moleküle liegen nicht isoliert, sondern in Form verschiedenster Komplexe vor. Dies betrifft sowohl Ionen, Atome und Moleküle im Blutkreislauf eines Patienten als auch in Blut, das zum Zwecke einer bestimmten Blut- bzw. Patientenbehandlung dem Patienten entnommen und außerhalb des Körpers mit geeigneten Komplexbildnern versetzt wird.

Ein Beispiel für die Komplexierung von Ionen ist die sogenannte citratantikoagulierte Hämodialyse/Hämofiltration. Hier erfolgt eine Komplexierung von $Ca^{++}$-Ionen des Blutes im extrakorporalen Blutkreislauf mittels Citrat, um während der Behandlung, vor allem während des Blut-Membran-Kontaktes im Dialysator, die Blutgerinnung zu hemmen.

Bei den meisten Hämodialysepatienten ist heute eine Hemmung der Blutgerinnung erforderlich. Standard ist die Verwendung von unfraktioniertem Heparin, das mit einer Spritzenpumpe in den arteriellen Schenkel des extrakorporalen Schlauchsystems infundiert wird. Die Verwendung sowohl von unfraktioniertem Heparin wie auch von alternativen Antikoagulantien wie niedermolekularem Heparin oder Hirudin ist für einen Teil der Patienten sowie auch für andere extrakorporale Bluttherapien aus den folgenden Gründen problematisch:

Die Antikoagulation wirkt systemisch (d. h. nicht nur beim Membrankontakt im extrakorporalen Kreislauf, sondern im ganzen Körper), was zu einer für einige Patienten kritischen Blutungsgefährdung führt. Insbesondere im intensivmedizinischen Bereich sind 30 - 40 % der Patienten blutungsgefährdet.

Einige Patienten zeigen Unverträglichkeitsreaktionen, wie z. B. die heparininduzierte Thrombozytopenie, die eine Verwendung dieser Antikoagulantien ausschließen.

Adsorptive Therapien (z. B. Leberersatztherapie) können mit einer Verwendung z. B. von Heparin inkompatibel sein, wenn dieses die (zur Adsorption von Toxinen vorgesehenen) Bindungsstellen des Adsorbers absättigt.

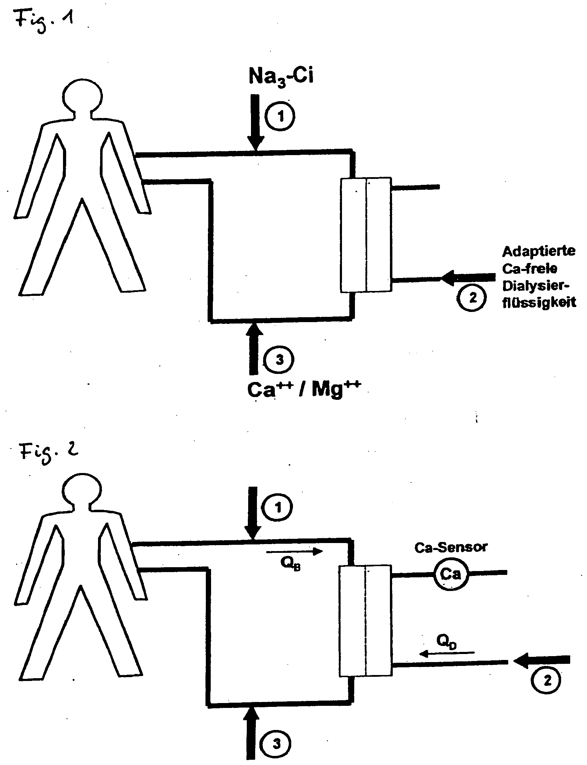

Eine Alternative, die die aufgezählten Probleme vermeidet, ist die regionale Gerinnungshemmung (nur im extrakorporalen Kreislauf, vor allem während des Blut-Membran-Kontakts) durch Citrat. Bei diesem, in Fig. 1 dargestellten Verfahren wird die Konzentration an freiem Calcium (Ca) durch Zugabe von Trinatrium-Citrat an der Stelle gemäß Pos. 1 in Fig. 1 soweit herabgesetzt, daß die Gerinnungskaskade unterbrochen wird. Zwei Citrat-Moleküle bilden dabei jeweils mit drei Ca-Ionen einen Komplex. Zur Konzentrationsreduktion trägt auch die Verwendung von Ca-freiem Dialysat gemäß Pos. 2 in Fig. 1 und der dadurch bedingte Ca-Entzug bei. Um eine Depletion des Körpers an Ca und Mg (welches ebenfalls von Citrat gebunden wird) zu vermeiden, muß eine weitere Lösung (siehe Pos. 3 in Fig. 1), welche Ca- und Mg-Ionen in adaptierter Konzentration enthält, in den venösen Schenkel des extrakorporalen Kreislaufs oder in einen separaten venösen Zugang infundiert werden.

Klinische Studien [Morita Y, Johnson RW, Dorn RE, Hall DS, "Regional anticoagulation during hemodialysis using citrate". The American Journal of the Medical Sciences (1961); Janssen MJMF et al., "Citrate compared to low molecular weight heparin anticoagulation in chronic hemodialysis patients", Kindney Int (1996) 49:806-813; Mehta RL, McDonald BR, Aguilar MM, Ward DM, "Regional citrate anticoagulation for continuous arteriovenous hemodialysis in critically ill patients". Kidney Int (1990) 38:976-981] weisen nach, daß die regionale Citrat-Antikoagulation sehr wirksam die Blutgerinnung im extrakorporalen Kreislauf verhindert. Gleichzeitig wird ein erhöhtes Blutungsrisiko für den Patienten vermieden. Die Citratdialyse gilt daher als interessante, wirkungsvolle Alternative zur herkömmlichen Heparin-Antikoagulation für jenen Teil der Patientenpopulation, bei der - wie oben beschrieben - die Verwendung von Heparin nachteilig oder klar kontraindiziert ist.

Trotz dieser klaren therapeutischen Vorteile wird die Citrat-Antikoagulation bisher nur in geringem Umfang, und nicht als automatisiertes, standardisiertes Verfahren eingesetzt. Die Gründe dafür sind:

1. Der erhöhte Aufwand: Bei der herkömmlichen Dialyse wird Standard-Dialysierflüssigkeit sowie eine geringe Menge Heparin benötigt. Bei der Citratdialyse werden üblicherweise drei Lösungen benötigt: Die Ca- und Mgfreie, in Na- und Bicarbonatgehalt adaptierte Dialysierflüssigkeit, die Trinatriumcitratlösung, und die $Ca^{++}/Mg^{++}$-Lösung.

2. Sicherheitsaspekte bei der Dosierung der $Ca^{++}/Mg^{++}$-Lösung: Wird hier falsch dosiert, kann schnell eine lebensbedrohliche Situation entstehen (Tetanie, Herzrhythmusstörungen, Herzstillstand). Eine falsche Dosierung kann sich aufgrund technischer Probleme (z. B. Ausfall der Pumpe, Lecks etc.) oder aber durch falsche Ermittlung des Ca-Bedarfs ergeben.

3. Konsequenzen einer Zufuhr von Citrat bzw. den $Ca_3$-$Ci_2$ bzw. $Mg_3$-$Ci_2$-Komplexen: Metabolische Alkalose, unphysiologische Ca-Konzentration.

Der Mehraufwand des Verfahrens kann durch geeignete technische Realisierung begrenzt werden; gewisse Mehrkosten sind bei Patienten mit Heparinunverträglichkeiten durchaus gerechtfertigt. Die Zufuhr von Citrat bzw. Citratkomplexen läßt sich durch effizienten Entzug über die Membran (Verwendung eines großflächigen Highflux-Filters, evtl. In Kombination mit postdilution-HDF) stark reduzieren. Eine geringe verbleibende Zufuhr ist wahrscheinlich tolerierbar; falls nicht, kann sie auch durch geeignete Modellierung des Dialyseprozesses und der Metabolisierung der Komplexe abgeschätzt und kompensiert werden.

Aus der WO 91/06326 ist ein citrat-antikoaguliertes Dialyseverfahren bekannt. Als Antikoagulant wird in der arteriellen Zufuhrleitung des extrakorporalen Blutkreislaufes Trinatriumcitrat zugegeben, wobei die zugegebene Menge an Trinatriumcitrat pro Zeiteinheit lediglich der Blutstromrate im extrakorporalen Kreislauf angepaßt wird. Bei einem Anstieg bzw. einer Verringerung der Blutstromrate wird die Zugaberate an Trinatriumcitrat ebenfalls erhöht bzw. erniedrigt. Die Calcium-Ionen-Konzentration des Patientenblutes wird hier nicht engmaschig überwacht, sondern in größeren Zeitabständen durch Blutproben ermittelt. Da eine engmaschige oder kontinuierliche Überwachung der CalciumKonzentration im Blut des Patienten fehlt und die Menge des zugegebenen Citrates sich lediglich nach der Blutflußrate und nicht nach dem Calciumlevel des Patienten orientiert, kann bei diesem vorbekannten Verfahren nicht sicher ausgeschlossen werden, daß sich Calcium-Werte im Blut des Patienten ergeben, die unphysiologisch sind und entsprechend lebensbedrohliche Konsequenzen für den Patienten nach sich ziehen können.

Zwar sind ionensensitive Sensoren bekannt, mittels derer die Calcium- oder Magnesium-Ionenkonzentration im Blut eines Patienten auch in kleinen Zeitintervallen ermittelt werden können. Jedoch ist die Verwendung eines blutseitigen Sensors wegen möglicher Toxizität, aufgrund von Sterilitätsanforderungen sowie aus Kostengründen nachteilig und daher unerwünscht.

Auch in anderen Anwendungsfällen als der Dialyse kann es notwendig sein, eine Konzentrationsermittlung von in Komplexen gebundenen Ionen, Atomen oder Molekülen vorzunehmen. Selbst unter der Annahme, daß ionensensitive Sensoren ohne Nachteile für den Patienten eingesetzt werden könnten, liefert deren Einsatz bei der Bestimmung der Konzentration von komplexierten Ionen oftmals keine oder nicht verwertbare Ergebnisse, da die Eigenschaften des in Komplex vorliegenden Ions eine sinnvolle Messung nicht zulassen.

Es ist die Aufgabe der vorliegenden Erfindung ein Verfahren zur Ermittlung der Konzentration eines in einem Komplex gebundenen Ions, Atoms oder Moleküls zu schaffen, mittels dessen zuverlässig die Konzentration des zu bestimmenden Ions, Atoms bzw. Moleküls ermittelt werden kann.

Diese Aufgabe wird durch ein Verfahren zur Ermittlung der Konzentration eines in einem Komplex gebundenen Ions, Atoms oder Moleküls gelöst, so, wie in den Ansprüchen definiert, bei dem zumindest während der Konzentrationsbestimmung durch Zugabe oder Entzug einer Substanz die Komplexbildung des Ions, Atoms oder Moleküls unterbunden wird. Der vorliegenden Erfindung liegt somit der Gedanke zugrunde, zum Zwecke der Konzentrationsermittlung die Bildung des Komplexes mit dem Ion, Atom oder Molekül zu vermeiden oder das Ion, Atom oder Molekül aus einem derartigen Komplex abzutrennen, um dann die entsprechende Konzentrationsermittlung vornehmen zu können.

Die zugegebene Substanz kann eine Säure sein. Die Komplexbildung wird in diesem Fall durch die mit der Zugabe der Säure einhergehende pH-Wert-Änderung unterbunden. Dabei wird der Komplexbildner protoniert und damit für die Komplexbildung der zu bestimmenden Substanz gleichsam inaktiviert.

Die Komplexbildung kann dadurch unterbunden werden, daß die Zugabe des Komplexbildners unterbrochen wird, das heißt dass hier die Zugabe der komplexbildenden Substanz beispielsweise durch Anhalten der sie fördernden Pumpe unterbrochen wird, oder der Komplexbildner mit einer anderen zuzugebenden Substanz einen Komplex eingeht und dabei das in seiner Konzentration zu ermittelnde Ion, Atom oder Molekül freigibt. Denkbar wäre somit die Zugabe einer Substanz, die zu dem Komplexbildner eine höhere Affinität aufweist als das zu bestimmende Ion, Atom oder Molekül. Letztere werden bei Zugabe dieser Substanz aus dem Komplex "herausgelöst" und stehen dann für eine Messung zur Verfügung. Bei der vorliegenden Erfindung handelt es sich um ein Verfahren zur Ermittlung der Ionenkonzentration des Blutes eines Patienten innerhalb eines extrakorporalen Blutkreislaufs bei der citrat-antikoagulierten Hämodialyse oder Hämodiafiltration. Erfindungsgemäß wird hierbei die Ionenkonzentration des Bluts aufgrund der Bestimmung der Ionenkonzentration im Dialysat ermittelt. Vor der Bestimmung der Ionenkonzentration im Dialysat wird die Komplexbildung des betreffenden Ions innerhalb des extrakorporalen Blutkreislaufs mit Citrat zum Zwecke der Konzentrationsermittlung unterbunden.

Dadurch läßt sich die Ionenkonzentration des Blutes eines Patienten kostengünstig und ohne Sicherheitsrisiken für den Patienten während der Behandlung überwachen.

Aufgrund der Tatsache, daß die Bestimmung der Ionenkonzentration nicht im Blut des Patienten, sondern im Dialysat erfolgt, ergibt sich der erhebliche Vorteil, daß entsprechend Sensoren zur Messung der Blut-Ionenkonzentration und die daraus resultierenden oben genannten Nachteile vermieden werden können. Ein Eingriff in den extrakorporalen Blutkreislauf ist daher zur Konzentrationsbestimmung nicht erforderlich. Entsprechendes gilt für die hier nicht notwendige wiederholte Entnahme von Blutproben.

Gemäß einer bevorzugten Ausgestaltung der vorliegenden Erfindung wird die Komplexbildung dadurch unterbunden, daß die Citrat-Zugabe in den Blutkreislauf vorübergehend unterbrochen wird. Da die blutseitige Konzentration im Dialysator nicht der Konzentration im Patienten entspricht, da die Ionen, insbesondere Ca-und Mg-Ionen, durch die Komplexbildung mit Citrat gebunden werden, wird nach dieser Ausführungsform die Citrat-Infusion unterbrochen, um die tatsächliche Ionenkonzentration an Ca oder Mg auch im Dialysator zu erhalten. Die Unterbrechung der Citrat-Zugabe bzw. -Infusion kann in regelmäßigen Intervallen erfolgen. Die Infusion der Ca- und Mg-Ionen kann in diesen Zeiträumen beibehalten werden, da die durch die Unterbrechung der Citratzugabe freigesetzten Ionen weitgehend im Dialysator dem Blut entzogen werden. Ebenso kann die Infusion der Ca- und Mg-Ionen reduziert werden, um den partiellen Entzug im Dialysator gerade zu kompensieren. Diese Überwachungsmethode ist diskontinuierlich, da die Citrat-Infusion nur für kurze Intervalle unterbrochen werden kann, um eine ausreichende Antikoagulation sicherzustellen. Dies genügt jedoch, um kritische Trends rechtzeitig zu erkennen. Bei Unter- oder Überdosierung oder Totalausfall der Ca/Mg-Ionen-Infusion dauert es nämlich einige Zeit, bis kritische Konzentrationen im Körper erreicht werden. Diese Zeit liegt bei einigen zehn Minuten, und hängt vor allem vom Blutfluß ab.

In weiterer Ausgestaltung der vorliegenden Erfindung ist vorgesehen, daß die Komplexbildung dadurch unterbunden wird, daß aus dem im Dialysat befindlichen Ion-Citrat-Komplex das Ion durch Absenkung des pH-Wertes freigesetzt wird. Die Konzentration des auf diese Weise freigesetzten Ions wird anschließend ermittelt. Eine derartige Ausführungsform der vorliegenden Erfindung hat den Vorteil, daß eine Unterbrechung der Citrat-Infusion hier nicht erforderlich ist. Setzt man das betreffende Ion, vorzugsweise Ca$^{++}$ und/oder Mg$^{++}$ aus dem Ion-Citrat-Komplex frei, läßt sich das entsprechende Ion im Dialysat ohne weiteres bestimmen. Das Verfahren ist jedoch verhältnismäßig komplex, da eine zusätzliche Infusion erforderlich ist, um die Freisetzung zu bewirken. Hier muß dialysatseitig Säure zugefügt werden. Hinzu kommt, daß der Ion-Citrat-Komplex mit einem Molekulargewicht von 504 (bei Ca-Ionen) relativ groß ist, so daß eine Clearance deutlich geringer als für Elektrolyte ist. Die Messung bzw. Abschätzung der blutseitigen aus der dialysatseitigen Konzentration gestaltet sich dann entsprechend schwieriger. Bei der Einstellung eines geeigneten pH-Wertes ist darauf zu achten, daß das Ca- bzw. Mg-Ion möglichst vollständig freigesetzt wird, da eine nur teilweise Freisetzung zu geringe Blutkonzentrationswerte ergeben würde.

In weiterer Ausgestaltung der vorliegenden Erfindung ist vorgesehen, daß nach Unterbrechung der Citrat-Zugabe die Messung der Ionenkonzentration im Dialysat nach Ablauf einer Zeitspanne durchgeführt wird, die sich aus einer durch Totvolumina bedingten Totzeit sowie einer zur Erreichung eines quasistationären Zustandes benötigten Zeitdauer zusammensetzt. Das Totvolumen ergibt sich aus den Volumina der Schlauchverbindung zwischen dem Infusionort für Citrat und dem Ort der Meßstelle für die entsprechende Ionen-Konzentration. Üblicherweise sind Totzeiten im Bereich von etwa 10 bis 30 s zu erwarten. Danach steigt die dialysatseitige Konzentration an. Dieser Anstieg ist nicht abrupt, sondern es ist mit Zeitkonstanten mit etwa 1 bis 2 min zu rechnen, bis ein quasistationärer Zustand erreicht wird. Darunter ist ein Zustand zu verstehen, ab dem Änderungen der Konzentrationen vernachlässigbar klein sind bzw. innerhalb einer vorgegebenen Toleranz liegen.

Gemäß einer bevorzugten Ausgestaltung der vorliegenden Erfindung ist vorgesehen, daß nach Unterbrechung der Citrat-Zugabe die Messung der Ionenkonzentration im Dialysat mehrfach wiederholt wird und der Meßwert bei Erreichen eines quasistationären Zustandes ermittelt wird. Man mißt somit zu mehreren Zeitpunkten die Konzentration im Dialysat, ermittelt auf diese Weise einen Konzentrationsverlauf und kann dann beurteilen, ob die Gleichgewichtskonzentration mit ausreichender Genauigkeit erreicht ist.

In weiterer Ausgestaltung der vorliegenden Erfindung ist vorgesehen, daß nach Unterbrechung der Citrat-Zugabe die Messung der Ionenkonzentration im Dialysat mehrfach wiederholt wird und der Meßwert durch Extrapolation der erhaltenen Ionenkonzentration im Dialysat ermittelt wird. Bei dieser Methode extrapoliert man somit den Gleichgewichtswert aus dem über ein ausreichendes Zeitintervall gemessenen Anstiegsverhalten der Konzentration im Dialysat. Diese Vorgehensweise weist den Vorteil auf, daß dadurch die Einstellung des Gleichgewichts nicht abgewartet werden muß. Jedoch ist hier eine Kenntnis des Zeitverhaltens des Konzentrationsanstiegs erforderlich.

Gemäß einer weiteren bevorzugten Ausgestaltung kann die Citrat-Konzentration für ein vorgegebenes Zeitintervall unterbrochen werden und der Meßwert wird durch Integration der Fläche der durch die Ionen-Konzentration im Dialysat als Funktion der Zeit definierten Antwortfunktion ermittelt. Die Citrat-Infusion kann beispielsweise für ein festes, relativ kurzes Zeitintervall, ca. 1 bis 2 min, unterbrochen werden. Durch Auswertung der Fläche unter pulsförmigen Antwortfunktion der Konzentration im Dialysat kann dann auf die Gleichgewichtskonzentration geschlossen werden.

Die Maßnahme der zeitweiligen Unterbrechung der Citrat-Infusion unterbricht selbstverständlich auch die regionale Antikoagulation im Dialysator. Jedoch ist nicht damit zu rechnen, daß sich daraus eine erhebliche Beeinträchtigung der Antikoagulation ergibt. So ist z. B. bei der sogenannten heparinfreien Dialyse, die eigentliche eine völlig antikoagulationsfreie Dialyse mit zyklischer Spülung des extrakorporalen Kreislaufes ist, die Komplikationsrate durch Blutgerinnung relativ gering (unter 5 %). Es ist daher nicht zu erwarten, daß eine zeitweise Unterbrechung der Antikoagulation, die sich dann insgesamt auf ca. 10 bis 20 % der Dialysedauer summieren dürfte, zu klinischen Problemen führt.

Wird eine Komplexbildung dadurch unterbunden bzw. aufgebrochen, daß aus dem im Dialysat befindlichen Ion-Citrat-Komplex das Ion durch Absenkung des pH-Wertes freigesetzt wird, wird vorzugsweise ein pH-Wert von 2 bis 3 eingestellt, da sich hier eine entsprechend vollständige Dissoziation des Komplexes realisieren läßt.

Die Einstellung eines pH-Wertes im Dialysat erfolgt vorzugsweise mittels einer Infusion von Säure.

Besonders vorteilhaft ist es, wenn zum Zwecke der Annäherung der Ionenkonzentration des Dialysats an die Ionenkonzentration des Blutes der Dialysatfluß reduziert wird. Durch Reduktion des Dialysatflusses wird die dialysatseitige Ionenkonzentration der blutseitigen Ionenkonzentration angeglichen.

Es wurde theoretisch und experimentell gezeigt, daß bei einer Abnahme des Dialysatflusses bei gleichbleibendem Blutfluß die Konzentration kleinmolekularer Substanzen sich immer mehr der Blut-Plasmakonzentration annähert. Wählt man den Dialysatfluß ausreichend klein - abhängig vom Blutfluß, dem interessierenden Molekül und dem verwendeten Dialysator - ist die Dialysatkonzentration praktisch identisch zur Blutkonzentration; das Dialysat erreicht dann eine Sättigung. Im Falle von Ionen wird die Dialysatkonzentration in diesem Zustand des Fließgleichgewichts jedoch wegen des Donnan-Effekts um einige Prozent von der Blut-Plasmakonzentration abweichen, was - bei Kenntnis oder Abschätzung des Plasmaproteingehalts - rechnerisch berücksichtigt werden kann.

In weiterer Ausgestaltung der vorliegenden Erfindung ist vorgesehen, daß die Ermittlung der Ionenkonzentration des Blutes ohne Reduzierung des Dialysatflußes durch Berechnung erfolgt. Eine derartige Vorgehensweise hat den Vorteil, daß der Dialysatfluß nicht erniedrigt werden muß und damit die Dialyseeffektivität in diesem Zeitintervall nicht reduziert wird.

Unter normalen Behandlungsbedingungen, bei denen der Dialysatfluß größer oder gleich dem Blutfluß ist, ergibt sich eine gegenüber der Blutkonzentration geringere Dialysatkonzentration. Bei Kenntnis der Dialysator-Transporteigenschaften für das betrachtete Molekül/Atom ($k_0A$), der Maschineneinstellungen (Blut- und Dialysatfluß) und des Hämatokrit läßt sich die Blut-Plasmakonzentration in guter Näherung aus der gemessenen dialysatseitigen Konzentration berechnen. Die blutseitige Konzentration berechnet sich bei Kenntnis der Clearance aus der einfachen Formel

$$C_{Bi} := (Q_D/K) * C_{Do}$$

Dabei ist $C_{Bi}$ die blutseitige Eingangskonzentration, $C_{Do}$ die gemessene dialysatseitige Ausgangskonzentration, $Q_D$ der Dialysatfluß und K die Clearance. K ist für eine in vitro-Situation mit rein diffusivem Transfer gegeben durch:

$$K := Q_B \cdot \frac{\exp\left[k0A \cdot \left(\frac{1}{Q_B} - \frac{1}{Q_D}\right)\right] - 1}{\exp\left[k0A \cdot \left(\frac{1}{Q_B} - \frac{1}{Q_D}\right)\right] - \frac{Q_B}{Q_D}}$$

Entsprechende komplexere Formalismen liegen auch für die in vivo Situation vor, ebenso für Hämodialyse mit Ultrafiltration, und für filtrative (HF) bzw. kombinierte filtrativ/diffusive (HDF) Verfahren. Dieser für jede beliebige Dialysesituation anwendbare mathematische Formalismus ist mit in vitro-und in vivo-Messungen validiert worden und steht in Form der Software "Clearance Calculation Tool CCT" von FMC in einer anwenderfreundlichen Form zur Verfügung.

Die Möglichkeit der Verringerung des Dialysatflußes ist zu bevorzugen, wenn eine genaue Messung erforderlich ist. Sie hat jedoch den Nachteil, daß für einige Minuten der Dialysatfluß erniedrigt werden muß und damit die Dialyseeffektivität in diesem Zeitintervall entsprechend reduziert ist. Sind für eine Überwachung der Ca-Ionen-Konzentration jedoch keine sehr genauen Messungen erforderlich, kann auch das Verfahren der Berechnung der Ionenkonzentration ohne Erniedrigung des Dialysatflußes angewandt werden, bei dem entsprechend keine Beeinflussung der Dialyse durch das Meßverfahren erfolgt, sieht man von der zeitweisen Unterbrechung der Antikoagulation ab.

Besonders vorteilhaft ist es, wenn die Erfassung der Ionenkonzentration im Dialysat mittels eines ionensensitiven Sensors in dem vom Dialysator abfließenden Dialysat erfolgt. Die Genauigkeit der Bestimmung der Ionenkonzentration des Blutes ist im wesentlichen durch das Sensorsystem gegeben. Da eine Fehlanzeige des Sensors in Kombination z. B. mit einer Fehldosierung ein hohes Gefahrenpotential für den Patienten darstellt, ist die Funktion des Sensors durch geeignete Prüfung oder andere Maßnahmen sicherzustellen. Eine wiederholte Funktionsprüfung des Sensors während der Dialyse kann erforderlich werden, wobei sich die Prüfintervalle an dem Zeitintervall orientieren, innerhalb dessen sich eine kritischer Zustand für den Patienten entwickeln kann (einige 10 min). Alternativ oder zusätzlich können redundante Sensorsysteme verwendet werden. Auch ist es möglich Sicherungssysteme vorzusehen, die ein charakteristisches Ausfallverhalten des Sensors sofort detektieren und zur Anzeige bringen. Weiterhin beschrieben ist, daß die ermittelte Ionenkonzentration des Blutes eines Patienten als Regelgröße dienen kann, deren Wert durch die Stellgrößen Citrat-Zugabe und/oder Zugabe eines ionenhaltigen Substitutionsmediums beeinflußt wird. Hier ist somit ein Regelkreis mit entsprechender Regeleinheit realisierbar, durch den ein gewünschter Wert der Ca-Ionen- und/oder Mg-Ionen-Konzentration im Patientenblut eingestellt werden kann. Auf diese Weise ist ein selbständig arbeitendes Überwachungssystem für die citrat-antikoagulierten Hämodialyse und/oder Hämofiltration realisierbar, durch das stets ein physiologischer Wert der Konzentration an Ca- und/oder Mg-Ionen im Blut des Patienten eingestellt werden kann. Als Stellgrößen können die Citrat-Zugabe bzw. die Zugabe des entsprechenden ionenhaltigen Substitutionsmediums dienen. Bei der Wahl der Citrat-Zugabe als Stellgröße ist jedoch zu beachten, daß diese bestimmte Grenzen nicht unterschreiten darf, um die Ionen-Konzentration im Bereich des Dialysators gering zu halten, was erforderlich ist, um die Koagulation wirksam zu vermeiden.

Eine besonders sichere Ausführung der folgenden Erfindung ergibt sich dadurch, daß ein Alarm ausgelöst wird, wenn die ermittelte Ionen-Konzentration im Blut des Patienten außerhalb eines zulässigen Bereiches liegt oder von einem zulässigen Wert abweicht. Ein Alarm kann ausgelöst werden, wenn eine Einzelmessung kritisch hohe oder niedrige Werte ergibt oder wenn ein kritischer Trend festgestellt wird. Zusätzlich oder alternativ zu einem Alarm können geeignete Gegenmaßnahmen eingeleitet werden, wie z. B. Bypass der Maschine, Anhalten der Infusionen, Änderung der Infusionsraten etc.

In weiterer Ausgestaltung der Erfindung ist vorgesehen, daß die Ionenkonzentration im blutseitigen Kompartiment des Dialysators ohne Unterbrechung der Citrat-Zufuhr bestimmt und mit einem zulässigen Grenzwert verglichen wird und in Abhängigkeit dieses Vergleiches die Citrat-Zufuhr verändert wird. Eine derartige Vorgehensweise ist sinnvoll, um zu prüfen, ob die Citrat-Zugabe ausreicht, um die Ca-Ionen-Konzentration soweit abzusenken, daß das Koagulationsrisiko auf das gewünschte Maß verringert wird. Diese ist selbstverständlich bei eingeschalteter Citrat-Zufuhr zu bestimmen, da der Überschuß des freien Ca bei Citrat-Zufuhr bestimmt werden muß. Hier ist ein regelndes System möglich, das automatisch die Citrat-Infusionsrate entsprechend der gemessenen Ca-Ionen-Konzentration variiert. Auch die erforderliche Ca-/Mg-Ionen-Substitution kann dadurch bestimmt werden.

Wie oben ausgeführt handelt es sich bei den Ionen vorzugsweise um Calcium-und/oder Magnesium-Ionen, die beide von Citrat in entsprechenden Komplexen gebunden werden. Vorzugsweise wird das Verfahren unter Ermittlung der Ca-Ionen-Konzentration im Dialysat durchgeführt. Ferner beschrieben ist ein Dialysegerät mit einem Hämodialysator und/oder Hämofilter sowie mit einem extrakorporalen Blutkreislauf, mit dem Mittel zur Zugabe von Citrat zum Blut stromaufwärts des Hämodialysators und/oder Hämofilters sowie Mittel zur Zugabe einer ionenhaltigen Substitutionslösung zum Blut stromabwärts des Hämodialysators und/oder Hämofilters in Verbindung stehen, sowie mit einer Dialysatleitung, die bezüglich der Fließrichtung des Dialysats stromabwärts des Hämodialysators und/oder Hämofilters Mittel zur Erfassung einer Ionenkonzentration im Dialysat aufweist. Die Mittel zur Erfassung der Ionenkonzentration sind vorzugsweise als einer oder mehrere ionensensitive Sensoren ausgeführt. Eine redundante Ausführung erhöht die Betriebssicherheit des Dialysegerätes und ermöglicht die rasche Erkennung von Fehlmessungen.

Eine sichere Ausführungsform des Dialysegerätes läßt sich dadurch realisieren, daß eine Prüfeinrichtung vorgesehen ist, die in Zeitintervallen oder bei Betätigung durch einen Bediener eine Funktionsüberprüfung des oder der Sensoren vornimmt. Mit der Dialysatleitung können Mittel zur Zugabe einer Substanz in Verbindung stehen, durch die der pH-Wert des Dialysats veränderbar ist. Auf diese Weise läßt sich das zu ermittelnde Ion nach dem Freisetzen aus dem Komplex durch pH-Wertänderung in seiner Konzentration ermitteln.

Die Mittel zur Zugabe der Substanz können derart angeordnet sein, daß die Zugabe bezüglich der Fließrichtung des Dialysats stromabwärts des Dialysators erfolgt.

Gemäß einer weiteren Ausgestaltung sind Mittel vorgesehen, durch die der Dialysatfluß vorübergehend verringerbar ist. Durch die Verringerung des Dialysatflusses läßt sich die Ionenkonzentration im Blut besonders genau aus der Ionenkonzentration im Dialysat berechnen.

Es kann eine Steuereinheit vorgesehen sein, die in Zeitintervallen oder bei Betätigung durch den Bediener die Mittel zur Zugabe von Citrat zum Blut derart ansteuert, daß die Zugabe vorübergehend unterbrochen wird, und die nach Beginn der Unterbrechung der Citrat-Zugabe den durch die Mittel zur Erfassung einer Ionenkonzentration im Dialysat ermittelten Konzentrationswert kontinuierlich oder in Zeitabständen aufzeichnet.

Die Steuereinheit kann derart ausgestaltet sein, daß diese den Meßwert der $Ca^{++}$-Ionenkonzentration nach einem Verfahren gemäß der Ansprüche 7 bis 10 ermittelt.

Gemäß einer weiteren bevorzugten Ausgestaltung ist eine Regeleinheit vorgesehen, die mit dem ionensensitiven Sensor sowie mit den Mitteln zur Zugabe von Citrat und/oder mit den Mitteln zur Zugabe einer ionenhaltigen Substitutionslösung verbunden ist, und die in Abhängigkeit des Vergleiches zwischen einem Sollwert oder Sollwertbereich und dem ermittelten Istwert der Ionenkonzentration eine Erhöhung oder der Zugabemenge an Citrat und/oder an ionenhaltiger Substitutionslösung veranlaßt. Auf diese Weise läßt sich die Konzentration im Blut des Patienten auf einen gewünschten Wert bzw. in einem gewünschten Intervall einregeln.

Die Regeleinheit und/oder die Mittel zur Zugabe von Citrat können derart ausgestaltet sein, daß die Konzentration an Citrat nicht unter einen Grenzwert absenkbar ist. Auf diese Weise wird verhindert, daß die Citrat-Konzentration nicht unter einen bestimmten Wert fällt, der für die wirksame Verhinderung der Koagulation erforderlich ist.

Es kann eine Alarmeinrichtung vorgesehen sein, die bei Ermittlung einer kritischen Einzelmessung der Ionenkonzentration oder bei Ermittlung eines kritischen Trends von Einzelmessungen einen Alarm auslöst. Der Bediener wird somit beispielsweise akustisch und/oder optisch auf einen derartigen kritischen Zustand hingewiesen.

Weitere Einzelheiten und Vorteile der vorliegenden Erfindung werden anhand eines in der Zeichnung dargestellten Ausführungsbeispiels näher erläutert. Es zeigen:

Fig. 1: eine schematische Darstellung eines Hämodialyseverfahrens mit Citrat-Antikoagulation mit verwendeten Lösungen und Position der Zuführung,

Fig. 2: eine schematische Darstellung gemäß Fig. 1 mit zusätzlicher Darstellung eines Ca-Ionen-Sensors in der vom Dialysator abführenden Dialysatleitung,

Fig. 3: eine Darstellung der Angleichung der dialysatseitigen Konzentration an die blutseitige Konzentration einer NaCl-Lösung bei Reduzierung des Dialysatflusses (in vitro-Experiment),

Fig.4: eine Darstellung der Simulation des zu erwartenden Konzentrationsverlaufs im Dialysat bei zeitweiliger Unterbrechung der Citrat-Infusion und

Fig. 5: eine schematische Darstellung eines Hämodialyseverfahrens mit Citrat-Antikoagulation; Ermittlung der Ca-Ionen-Konzentration durch Dissoziation der Ca-Citrat-Komplexe im Dialysat.

Fig. 1 zeigt in schematischer Darstellung ein Hämodialyseverfahren mit Citrat-Antikoagulation. Durch die Zugabe von Trinatriumcitrat (siehe Pos. 1) in dem vom Patienten zum Dialysator führenden Teil des extrakorporalen Kreislaufs wird die Konzentration an freien Ca-Ionen soweit herabgesetzt, daß die Gerinnungskaskade unterbrochen wird. Es entsteht ein Tricalcium-Bicitratkomplex. Eine weitere Konzentrationsreduktion von Ca-Ionen im Blut wird dadurch herbeigeführt, daß Cafreies Dialysat (siehe Pos. 2) verwendet wird, so daß sich ein entsprechend hoher Gradient der Ca-Ionen-Konzentration über die Membran ergibt. Um eine unzulässige Anreicherung von Calcium und auch Magnesium, welches ebenfalls von Citrat gebunden wird, im Körper des Patienten zu vermeiden, wird eine Substitutionslösung (siehe Pos. 3), die Ca- und Mg-Ionen in adaptierter der Konzentration enthält, in den venösen Schenkel des extrakorporalen Kreislaufs infundiert. Die entsprechende Substitutionslösung kann selbstverständlich auch in einen separaten venösen Zugang des Patienten infundiert werden.

Zur Durchführung des erfindungsgemäßen Verfahrens wird die Komplexbildung des Ca-Citrat-Komplexes beispielsweise dadurch unterbunden, daß die Zugabe von Citrat vorübergehend unterbrochen wird. Entsprechend steigt die Ca-Konzentration an und es kann mit dem in Fig. 2 ersichtlichen Ca-Sensor dialysatseitig die Ca-Ionen-Konzentration gemessen werden. Die Größen $Q_D$ und $Q_B$ kennzeichnen den Dialysat- bzw. Blutfluß. Eine Bestimmung der Ca-Ionen-Konzentration im Patientenblut ist dadurch möglich, daß der Dialysatfluß soweit reduziert wird, daß die dialysatseitige Ca-Ionen-Konzentration der blutseitigen Ca-Ionen-Konzentration angeglichen wird. Ohne Änderung des behandlungsüblichen Blut- und Dialysatflusses kann die blutseitige Konzentration aus der gemessenen dialysatseitigen Konzentration auch mittels der bekannten oben genannten Beziehungen zwischen der blutseitigen Eingangskonzentration der Ca-Ionen, der gemessenen dialysatseitigen Ausgangskonzentration der Ca-Ionen, dem Dialysatfluß sowie der Clearance bestimmt werden.

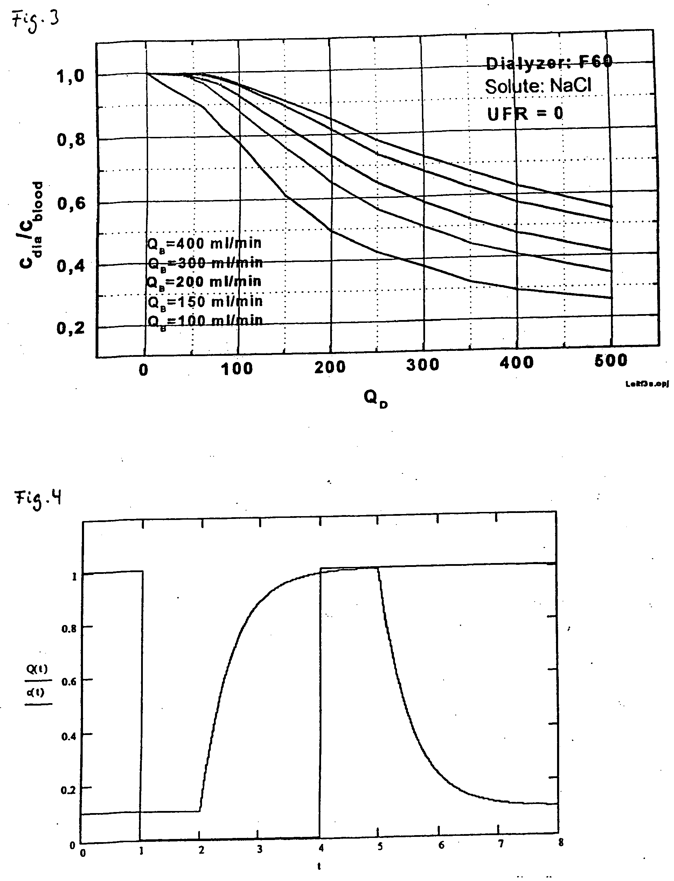

Fig. 3 zeigt die Angleichung der dialysatseitigen Konzentration einer NaCl-Lösung an die blutseitige Konzentration bei Reduzierung des Dialysatflusses. Wählt man den Dialysatfluß ausreichend klein - abhängig vom Blutfluß, dem betreffenden Molekül und dem verwendeten Dialysator - entspricht die Dialysatkonzentration praktisch der Blutkonzentration. Das Dialysat erreicht dann eine Sättigung. Auf diese Weise läßt sich aus der gemessenen Konzentration im Dialysat mit guter Genauigkeit die Konzentration im Blut bestimmen.

Fig. 4 zeigt die Antwortfunktion der Ca-Ionen-Konzentration im Dialysat bei sprunghafter Unterbrechung der Citrat-Infusion. Die dargestellte Rechteckfunktion gibt den Zeitverlauf der Citrat-Infusion wieder. Wie aus Fig. 4 ersichtlich, ist die Citrat-Infusion im Zeitintervall t = 1 bis t = 4 unterbrochen. Die andere dargestellte Kurve gibt den dialysatseitigen Konzentrationsverlauf an Ca-Ionen wieder. Die Ordinaten beider Figuren sowie die Abszisse haben beliebige Einheiten, da es hier auf Absolutwerte nicht ankommt. Nach dem Abschalten des Infusionsflusses (t = 1) wird zunächst am Ort des Sensors noch die niedrige Ca-Ionen-Konzentration bestehenbleiben. Grund ist, daß das Volumen der Schlauchverbindungen zwischen Infusionsort und dem Ort des Sensors als Totvolumen wirkt. Üblich sind Totzeiten im Bereich von etwa 10 bis 30 s. Wie aus Fig. 4 weiter ersichtlich, steigt anschließend die dialysatseitige Ca-Ionen-Konzentration an. Wegen der Vermischung von Blut und Dialysat jeweils mit hoher und niedriger Citrat-Konzentration vor allem im Dialysat wird der Anstieg nicht scharf sein, sondern die Konzentration wird sich an einen Gleichgewichtswert annähern. Der Anstieg ist in Fig. 4 vereinfachend als Exponentialfunktion dargestellt. Üblicherweise ist hiermit Zeitkonstanten von etwa 1 bis 2 min zu rechnen. Danach ist ein quasistationärer Zustand erreicht, so daß die hier ermittelte Konzentration mit guter Genauigkeit als Meßwert dienen kann. Nach Wiedereinschalten der Citrat-Infusion (t = 4) wird zunächst wieder die Totzeit wirksam. Anschließend sinkt die Ca-Ionen-Konzentration ab, um sich schließlich dem niedrigen Gleichgewichtswert bei laufender Citrat-Infusion anzunähern.

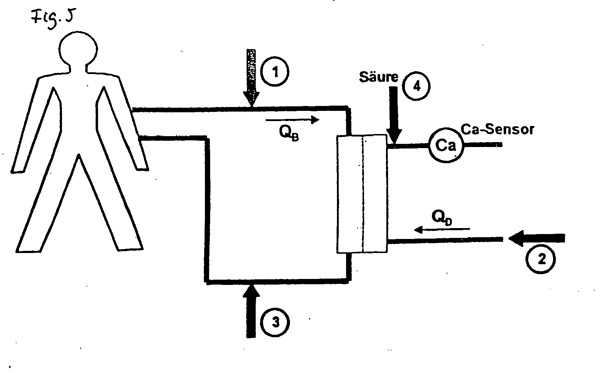

Fig. 5 zeigt eine Verfahrensvariante, bei der die Unterbrechung der Citrat-Infusion nicht erforderlich ist. Hier wird die Ca-Ionen-Konzentration dadurch bestimmt, daß aus dem Ca-Citrat-Komplex das Ca-Ion wieder freigesetzt wird. Dies geschieht durch Zufuhr einer Säure (siehe Pos. 4). Hierdurch wird der pH-Wert vorzugsweise auf einem Bereich von 2 bis 3 reduziert, was zu einer Dissoziation des Komplexes führt und die Ca-Ionen entsprechend freisetzt. Vorteil dieser Verfahrensvariante ist es, daß eine Unterbrechung der Antikoagulation nicht erfolgt, da die Citrat-Zugabe nicht unterbrochen wird.

Die vorliegende Erfindung ermöglicht es, den eigentlichen physiologisch relevanten und kritischen Parameter der Ca-Ionen- bzw. Mg-Ionen-Konzentration des Patientenblutes während der gesamten Therapie zu überwachen, so daß zu jedem Zeitpunkt der Behandlung sichergestellt ist, daß ein die Patientengesundheit gefährdender Zustand ausgeschlossen ist. Voraussetzung ist selbstverständlich, daß die Ermittlung des Ca- bzw. Mg-Ionen-Wertes zuverlässig erfolgt. Hier kann durch eine redundante Sensorausführung oder durch Sensoren, die Selbsttests durchführen eine gute Zuverlässigkeit erreicht werden. Neben dem Einsatz von Sensoren sind selbstverständlich auch andere Methoden zur Ermittlung der Ionen-Konzentration einsetzbar.

Während vorbekannte Überwachungssysteme im allgemeinen nur einzelne technische Komponenten betreffen, wie z. B. die Überwachung einer Pumpe mit separaten oder integrierten Überwachungssystemen, z. B. Tropfenzählern, die die Überwachung einer Infusion gestatten, schließt die erfindungsgemäße Überwachung der Ionenkonzentration des Patienten als den eigentlichen physiologisch relevanten kritischen Parameter die gesamte Therapie ein und berücksichtigt damit jeden Teilaspekt des Verfahrens.

## Patentansprüche

1.  Verfahren zur Ermittlung der Ionenkonzentration des Blutes eines Patienten innerhalb eines extrakorporalen Blutkreislaufs eines Dialysegerätes bei der citrat-antikoagulierten Hämodialyse oder Hämodiafiltration, **dadurch gekennzeichnet,**

    **dass** die Ionenkonzentration des Blutes aufgrund der Bestimmung der Ionenkonzentration im Dialysat ermittelt wird, und
    **dass** vor der Bestimmung der Ionenkonzentration im Dialysat die Komplexbildung des betreffenden Ions innerhalb des extrakorporalen Blutkreislaufs mit Citrat zum Zwecke der Konzentrationsermittlung unterbunden wird.

2.  Verfahren nach einem der vorhergehenden Ansprüche, **dadurch gekennzeichnet, dass** die Komplexbildung dadurch unterbunden wird, dass die Citrat-Zugabe in den Blutkreislauf vorübergehend unterbrochen wird.

3.  Verfahren nach Anspruch 2, **dadurch gekennzeichnet, dass** nach Unterbrechung der Citrat-Zugabe die Messung der Ionenkonzentration im Dialysat nach Ablauf einer Zeitspanne durchgeführt wird, die sich aus einer durch Totvolumina bedingten Totzeit sowie einer zur Erreichung eines quasistationären Zustandes benötigten Zeitdauer zusammensetzt.

4.  Verfahren nach Anspruch 2, **dadurch gekennzeichnet, dass** nach Unterbrechung der Citrat-Zugabe die Messung der Ionenkonzentration im Dialysat mehrfach wiederholt wird und der Messwert bei Erreichen eines quasistationären Zustandes ermittelt wird.

5.  Verfahren nach Anspruch 2, **dadurch gekennzeichnet, dass** nach Unterbrechung der Citrat-Zugabe die Messung der Ionenkonzentration im Dialysat mehrfach wiederholt wird und der Messwert durch Extrapolation der erhaltenen Ionenkonzentrationen im Dialysat ermittelt wird.

6.  Verfahren nach Anspruch 2, **dadurch gekennzeichnet, dass** die Citrat-Konzentration für ein vorgegebenes Zeitintervall unterbrochen wird und der Messwert durch Integration der Fläche der durch die Ionenkonzentration im Dialysat als Funktion der Zeit definierten Antwortfunktion ermittelt wird.

7.  Verfahren nach Anspruch 1, **dadurch gekennzeichnet, dass** die Komplexbildung dadurch unterbunden wird, dass aus dem im Dialysat befindlichen Ion-Citrat-Komplex das Ion durch Absenkung des pH-Wertes freigesetzt wird.

8.  Verfahren nach Anspruch 7, **dadurch gekennzeichnet, dass** der pH-Wert auf den Bereich pH=2-3 eingestellt wird.

9. Verfahren nach Anspruch 7 oder 8, **dadurch gekennzeichnet, dass** die Einstellung des pH-Wertes im Dialysat mittels einer Infusion von Säure erfolgt.

10. Verfahren nach einem der vorhergehenden Ansprüche, **dadurch gekennzeichnet, dass** zum Zwecke der Annäherung der Ionenkonzentration des Dialysats an die Ionenkonzentration des Blutes der Dialysatfluss reduziert wird.

11. Verfahren nach einem der vorhergehenden Ansprüche, **dadurch gekennzeichnet, dass** die Ermittlung der Ionenkonzentration des Blutes ohne Reduzierung des Dialysatflusses durch Berechnung erfolgt.

12. Verfahren nach einem der vorhergehenden Ansprüche, **dadurch gekennzeichnet, dass** die Erfassung der Ionenkonzentration im Dialysat mittels eines ionensensitiven Sensors in dem vom Dialysator abfließenden Dialysat erfolgt.

13. Verfahren nach einem der vorhergehenden Ansprüche, **dadurch gekennzeichnet, dass** ein Alarm ausgelöst wird, wenn die ermittelte Ionenkonzentration im Blut des Patienten außerhalb eines zulässigen Bereiches liegt oder von einem zulässigen Wert abweicht.

14. Verfahren nach einem der vorhergehenden Ansprüche, **dadurch gekennzeichnet, dass** die Ionenkonzentration im blutseitigen Kompartiment des Dialysators ohne Unterbrechung der Citrat-Zufuhr bestimmt und mit einem zulässigen Grenzwert der Ionenkonzentration verglichen wird und in Abhängigkeit dieses Vergleichs die Citrat-Zufuhr verändert wird.

15. Verfahren nach einem der vorhergehenden Ansprüche, **dadurch gekennzeichnet, dass** es sich bei den Ionen um Calcium- und/oder Magnesium-Ionen handelt.

## Claims

1. A method of determining the ion concentration of the blood of a patient within an extracorporeal blood circuit of a dialysis machine in hemodialysis or hemodiafiltration anti-coagulated with citrate,
   **characterized in that**
   the ion concentration of the blood is determined on the basis of the detection of the ion concentration in the dialysate; and
   **in that** the complex formation of the respective ion within the extracorporeal blood circuit using citrate is suppressed for the purpose of determining the concentration prior to the detection of the ion concentration in the dialysate.

2. A method in accordance with one of the preceding claims, **characterized in that** the complex formation is suppressed **in that** the citrate addition into the blood circuit is temporarily interrupted.

3. A method in accordance with claim 2, **characterized in that** the measurement of the ion concentration in the dialysate is carried out after the interruption of the citrate addition after the elapse of a time period that is composed of a dead time caused by the dead volumes and of a time period required to achieve a quasi-stationary state.

4. A method in accordance with claim 2, **characterized in that** the measurement of the ion concentration in the dialysate is repeated a multiple of times after interruption of the citrate addition and the measured value on the achieving of a quasi-stationary state is determined.

5. A method in accordance with claim 2, **characterized in that** the measurement of the ion concentration in the dialysate is repeated a multiple of times after interruption of the citrate addition and the measured value is determined by extrapolation of the obtained ion concentrations in the dialysate.

6. A method in accordance with claim 2, **characterized in that** the citrate concentration is interrupted for a predefined time interval and the measured value is determined by integration of the area of the response function defined by the ion concentration in the dialysate as a function of time.

7. A method in accordance with claim 1, **characterized in that** the complex formation is suppressed **in that** the ion is released from the ion citrate complex present in the dialysate by lowering the pH.

8. A method in accordance with claim 7, **characterized in that** the pH is set to a range pH = 2-3.

9. A method in accordance with claim 7 or claim 8, **characterized in that** the setting of the pH in the dialysate takes place by means of an infusion of acid.

10. A method in accordance with one of the preceding claims, **characterized in that** the dialysate flow is reduced for the purpose of approximating the ion concentration of the dialysate to the ion concentration of the blood.

11. A method in accordance with one of the preceding claims, **characterized in that** the determination of the ion concentration of the blood takes place by calculation without reducing the dialysate flow.

12. A method in accordance with one of the preceding claims, **characterized in that** the detection of the ion concentration in the dialysate takes place by means of an ion-sensitive sensor in the dialysate flowing off from the dialyser.

13. A method in accordance with one of the preceding claims, **characterized in that** an alarm is triggered when the determined ion concentration in the blood of the patient is outside a permitted range or differs from a permitted value.

14. A method in accordance with one of the preceding claims, **characterized in that** the ion concentration in the blood-side compartment of the dialyser is determined without interruption of the citrate supply and is compared with a permitted limit value of the ion concentration and the citrate supply is changed in dependence on this comparison.

15. A method in accordance with one of the preceding claims, **characterized in that** the ions are calcium ions and/or magnesium ions.

## Revendications

1. Méthode pour la détermination de la concentration des ions dans le sang d'un patient à l'intérieur d'un circuit sanguin extracorporel d'un appareil de dialyse lors de l'hémodialyse ou l'hémodiafiltration à anticoagulation au citrate, **caractérisée en ce que**
la concentration des ions dans le sang est déterminée sur la base de la détermination de la concentration des ions dans le dialysat, et
**en ce que**, avant la détermination de la concentration des ions dans le dialysat, la complexation de l'ion concerné à l'intérieur du circuit sanguin extracorporel avec du citrate est empêchée à des fins de détermination de la concentration.

2. Méthode selon l'une des revendications précédentes, **caractérisée en ce que** la complexation est empêchée par le fait que l'addition de citrate dans le circuit sanguin est temporairement interrompue.

3. Méthode selon la revendication 2, **caractérisée en ce que**, après l'interruption de l'addition de citrate, la mesure de la concentration des ions dans le dialysat est effectuée après écoulement d'un laps de temps, qui se compose d'un temps mort inhérent à des volumes morts ainsi que d'une durée nécessaire à l'obtention d'un état presque stationnaire.

4. Méthode selon la revendication 2, **caractérisée en ce que**, après l'interruption de l'addition de citrate, la mesure de la concentration des ions dans le dialysat est répétée plusieurs fois et la valeur de mesure est déterminée lors de l'obtention d'un état presque stationnaire.

5. Méthode selon la revendication 2, **caractérisée en ce que**, après l'interruption de l'addition de citrate, la mesure de la concentration des ions dans le dialysat est répétée plusieurs fois et la valeur de mesure est déterminée par extrapolation des concentrations des ions dans le dialysat obtenues.

6. Méthode selon la revendication 2, **caractérisée en ce que** la concentration de citrate est interrompue pour un intervalle de temps prédéfini et la valeur de mesure est déterminée par intégration de la surface de la fonction de réponse définie par la concentration des ions dans le dialysat en fonction du temps.

7. Méthode selon la revendication 1, **caractérisée en ce que** la complexation est empêchée par le fait que l'ion est libéré du complexe ion-citrate présent dans le dialysat par diminution de la valeur de pH.

8. Méthode selon la revendication 7, **caractérisée en ce que** la valeur de pH est réglée dans la plage de pH=2-3.

9. Méthode selon la revendication 7 ou 8, **caractérisée en ce que** le réglage de la valeur de pH dans le dialysat est effectué au moyen d'une perfusion d'acide.

10. Méthode selon l'une des revendications précédentes, **caractérisée en ce que** le flux de dialysat est réduit afin de rapprocher la concentration des ions dans le dialysat de la concentration des ions dans le sang.

11. Méthode selon l'une des revendications précédentes, **caractérisée en ce que** la détermination de la concentration des ions dans le sang est effectuée par calcul sans réduction du flux de dialysat.

12. Méthode selon l'une des revendications précédentes, **caractérisée en ce que** la détection de la concentration des ions dans le dialysat est effectuée au moyen d'un capteur ionosensible dans le dialysat sortant du dialyseur.

13. Méthode selon l'une des revendications précédentes, **caractérisée en ce qu'**une alarme est déclenchée quand la concentration déterminée des ions dans le sang du patient se trouve hors d'une plage autorisée ou diffère d'une valeur autorisée.

14. Méthode selon l'une des revendications précédentes, **caractérisée en ce que** la concentration des ions dans le compartiment côté sang du dialyseur est déterminée sans interruption de l'alimentation en citrate et est comparée à une valeur limite autorisée de la concentration des ions, et l'alimentation en citrate est modifiée en fonction de cette comparaison.

15. Méthode selon l'une des revendications précédentes, **caractérisée en ce que** les ions sont des ions calcium et/ou des ions magnésium.

Fig. 1

Na$_3$-Ci

① 

② Adaptierte Ca-freie Dialysier- flüssigkeit

③ 

Ca$^{++}$ / Mg$^{++}$

Fig. 2

① 

$Q_B$

Ca-Sensor
Ca

$Q_D$

② 

③

Fig.3

Fig.4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9106326 A


**In der Beschreibung aufgeführte Nicht-Patentliteratur**

- **MORITA Y ; JOHNSON RW ; DORN RE ; HALL DS.** Regional anticoagulation during hemodialysis using citrate. The American Journal of the Medical Sciences, 1961
- **JANSSEN MJMF et al.** Citrate compared to low molecular weight heparin anticoagulation in chronic hemodialysis patients. *Kindney Int,* 1996, vol. 49, 806-813
- **MEHTA RL ; MCDONALD BR ; AGUILAR MM ; WARD DM.** Regional citrate anticoagulation for continuous arteriovenous hemodialysis in critically ill patients. *Kidney Int,* 1990, vol. 38, 976-981